# EUROPEAN PATENT APPLICATION

(11) **EP 1 484 075 A1**
(43) Date of publication of application: **08.12.2004**
(21) Application number: 04013026.2
(22) Date of filing: 02.06.2004
(51) Int. Cl.: A61M 16/06, A61M 25/02

(54) **Tube seal adaptor for respiratory masks**

(30) Priority: 03.06.2003 US 453799
(71) Applicant: Hans Rudolph, Inc., Kansas City, Missouri 64114 (US)
(72) Inventor: Landis, Robert M., Mountainside, NJ 07092 (US)
(74) Representative: Grünecker, Kinkeldey, Stockmair & Schwanhäusser Anwaltssozietät

(57) **Abstract**

A tube seal adaptor (10) for forming a seal around a tube (2) inserted between a respiratory mask (6) and the face (4) of the mask wearer comprises a curved strip of flexible and pliable material having a tacky base (14) and a slotted curved outer surface (16) which curves downward to a relatively sharp angle with the ends (22,24) of the strip. A slot (27) extends into the curved strip from an outer surface thereof medially between the ends. The slot is sized to receive a feeding tube or the like in such a manner that the material forming the adaptor will seal around the tube when a mask is pressed or pulled against the seal on the wearer's face.

## Description

### Background of the Invention

The present invention relates to seals for respiratory masks inducting masks covering the mouth or nose or both. The present invention is particularly adapted for use with respiratory masks in situations in which feeding tubes, drug delivery tubes or other types of catheters extend into a patients nose or mouth while the respiratory mask is in place delivering oxygen or other gasses to the wearer.

Most respiratory masks have integrally formed seals in the form of flexible or pliable flanges, cushions, pillows or the like extending around their outer periphery to form a seal between the mask and the face of the wearer. See for example the masks shown and described in U.S. Patent Nos. 5,265,595 and 6,192,886. The seals prevent air from leaking through the interface between the mask and the wearer's face.

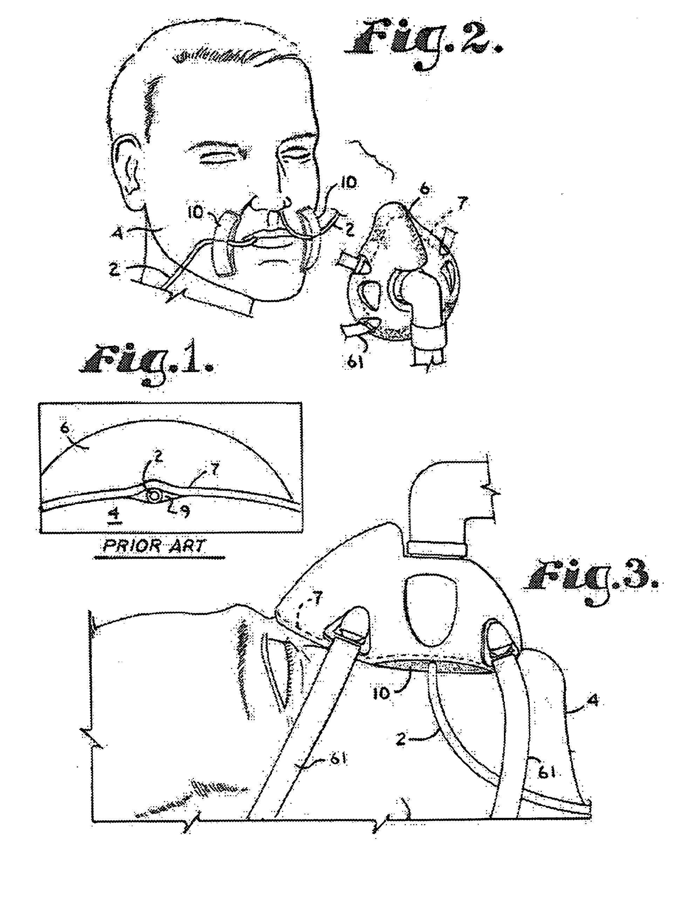

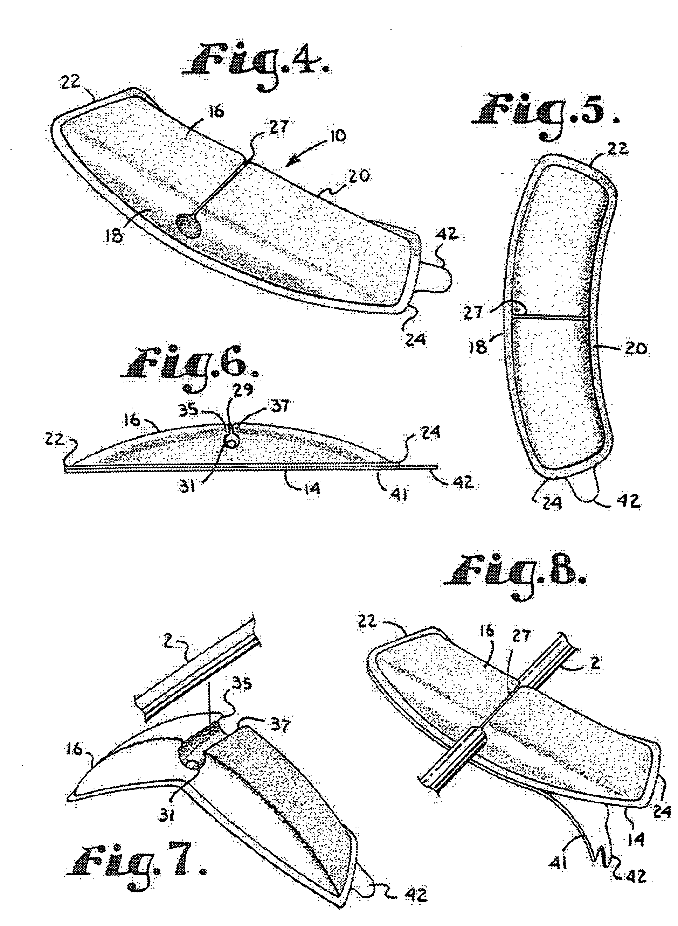

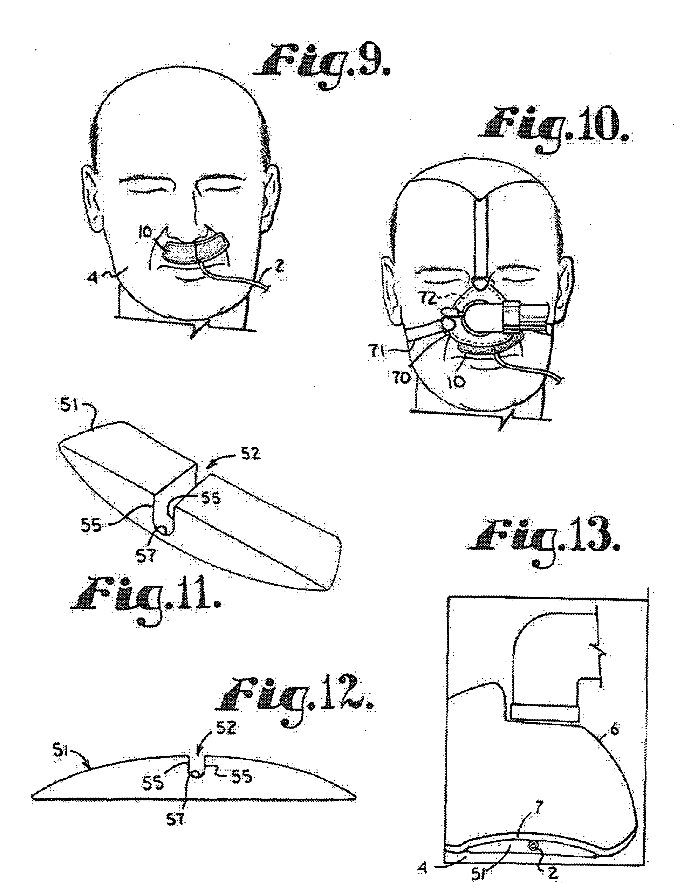

The extension of tubes between the face of a wearer and the associated seal of the mask creates openings between the mask and the face of the wearer around the tube which permits gas to escape therethrough. See for example Figure 1 (labeled Prior Art) which is a cross-sectional view showing a tube 2 positioned between a patient's face 4 and a mask 6. Flexible flange 7 on the mask 6 conforms somewhat to the upper surface of the tube 2 but cannot conform completely to the shape of the tube 2 thereby leaving gaps or openings 9 between the mask flange 7 and the patient's face 4. Pressurized gas, escaping or leaking through such openings 9, generates noise and can generate a tickling sensation on the face of the wearer which can be annoying. In addition, such leaks may adversely affect any breath analysis being performed on the patient using the respiratory mask or may result in an inadequate volume or concentration of gasses being delivered to a patient.

Although numerous anchoring systems have been developed for fixing the relative position of a tube or catheter on a patient's face or in association with a mask, such anchoring systems are not designed to for use in association with respiratory masks in a manner that prevents the respiratory gasses from leaking out around a tube inserted between a mask and the wearer's face.

### Summary of the Invention

The tube seal adaptor of this invention generally comprises an arcuate strip or patch of pliable material with a convex outer surface and a tube receiving notch or slot formed in the strip generally at the thickest portion thereof. An inner surface or base of the adaptor is sticky or tacky to permit the inner surface to be secured to the face of a wearer. The adaptor is positioned on the face at a location corresponding to the location where a sealing member of the mask would normally abut against the face of the wearer and where a tube extending through the adaptor may be readily directed to the mouth or desired nasal passage.

The notch or slot may be of various geometries. Preferred geometries include a slot with parallel sides and a rounded lower edge, or a notch including a reduced opening entry which opens into a larger or wider cylindrical groove or bore extending lengthwise across the strip. A tube, such as a feeding tube or drug delivery tube, is positioned in the cylindrical groove or the slot and then the mask may be positioned on the wearer's face with a portion of the mask extending across the adaptor to form a seal therebetween.

The force of the mask pressing against the face of the wearer and the adaptor, causes the pliable adaptor to form a seal around the tube positioned in the slot or groove therein. In the embodiment incorporating a cylindrical groove with a reduced opening entry, the cylindrical groove of the adaptor selected preferably has a diameter approximately the same size as or slightly smaller than the outer diameter of the tube to be used therewith. The edges of the reduced opening entry, extending above the tube in the cylindrical groove are pressed together by the compression of the mask against the adaptor to form a complete seal around the tube.

In the embodiment incorporating a relatively straight walled slot, the depth of the slot is at least slightly greater than the radius of the tube and preferably approximately twice as large as the outer diameter of the tube.

### Brief Description of the Drawings

Figure 1, labeled prior art, is a cross-sectional view showing a tube inserted between a sealing flange of an oro-nasal mask and the face of the mask wearer without the tube seal adaptor of the present invention.
Figure 2 is an exploded and fragmentary perspective view showing a pair of tube seal adaptors of the present invention positioned between an oro-nasal mask and the face of a mask wearer and supporting tubes extending into the wearer's mouth and nose.
Figure 3 is a fragmentary and enlarged side view showing a tube extending through a tube seal adaptor of the present invention positioned between an oro-nasal mask and the wearer's face.
Figure 4 is an enlarged perspective view of the tube seal adaptor of the present invention.
Figure 5 is a top plan view of the tube seal adaptor of the present invention.
Figure 6 is a left side elevational view of the tube seal adaptor of the present invention.
Figure 7 is a perspective view of the tube seal adaptor showing a tube being inserted in a tube receiving notch formed therein.
Figure 8 is a perspective view of the tube seal adaptor having a tube secured within the tube receiving notch and showing a protective backing sheet being removed from a sticky base of the adaptor.
Figure 9 is a view showing one of the tube seal adaptors secured to the upper lip of a wearer for supporting a tube extending into a nostril of a wearer.
Figure 10 is a view similar to Figure 9 showing a nasal mask secured over the tube seal adaptor.
Figure 11 is a perspective view of an alternative embodiment of the tube seal adaptor of the present invention.
Figure 12 is a left side elevational view of the alternative embodiment of the tube seal adaptor.
Figure 13 is a view similar to Figure 1 showing a tube secured in a notch of the alternative embodiment of the tube seal adaptor which is positioned between the sealing flange of a mask and the face of the mask wearer.

### Detailed Description of the Invention

As required, detailed embodiments of the present invention are disclosed herein; however, it is to be understood that the disclosed embodiments are merely exemplary of the invention, which may be embodied in various forms. Therefore, specific structural and functional details disclosed herein are not to be interpreted as limiting, but merely as a basis for the claims and as a representative basis for teaching one skilled in the art to variously employ the present invention in virtually any appropriately detailed structure.

Referring to the drawings in more detail, and in particular Figures 2-10, there is shown a first embodiment of a tube seal adaptor or seal 10 of the present invention, for use in forming a seal around an oro-nasal tube 2 which is intended to be inserted between a mask 6 and the face 4 of the mask wearer. As used herein, the term oro-nasal tube is intended to include any feeding tube, drug delivery tubes, nasal gastric tubes, or catheters such as an esophageal balloon catheter which might be inserted between an oro-nasal respiratory mask and the face of a mask wearer. The adaptor 10 is designed to form a seal around the tube and between the mask 6 and the face 4 of the mask wearer. The mask 6, shown in Figures 2 and 3 is an oro-nasal mask adapted to cover the mouth and nose of a wearer and is the type having an inwardly directed flexible sealing flange 7 as generally shown in Figure 11. It is to be understood that the tube seal adaptor is adapted for use with masks including means for sealing other than a flexible sealing flange, including air, gel or foam filled cushions, pillows or the like.

Figures 4-8 show details of a first embodiment of the tube seal adaptor 10. The adaptor 10 is formed from a soft, flexible and pliable material such as a closed cell foam. Preferred foams for use include dosed cell cross-linked polyolefins, such as: ethylene vinyl acetate (EVA); low density polyethylene (LDPE); high density polyethylene (HDPE) and polypropylene (PP). The adaptor 10 is preferably formed in the shape of an arcuate strip, having a base 14, a convex outer surface 16, a first arcuate side 18, a second arcuate side 20, a first end 22 and a second end 24. However, it is foreseen that other configurations of the adaptor could be utilized, and such a configuration might more appropriately be referred to as a patch instead of a strip.

The adaptor 10 is thickest toward its middle and the outer surface 16 tapers downward toward the ends 22 and 24 where it comes to a relatively sharp edge. The sides 18 and 20 of the adaptor 10 are curved to generally conform to the curved outer edge of the mask 6 with which it is to be used and to conform to the shape of the inner flange 7 extending along the cheek of a mask wearer proximate the edge of the patient's mouth.

A notch 27 is formed in the arcuate strip generally medially between the first and second ends 22 and 24 in the thickest portion of the adaptor 10. The notch 27 extends inward from the outer surface 16 of the adaptor 10 toward the base 14 and extends across the strip between the first and second arcuate sides 18 and 20. The notch 27 is sized to receive an oro-nasal tube and the notch 27 is at least as deep as it is wide at its widest dimension, but is preferably approximately twice as deep as it is wide.

Referring to Figures 4 and 6, the notch 27 shown therein includes a reduced throat section 29 which extends inward from the outer surface 16 to a cylindrical section 31. As discussed above, the notch 27 is preferably twice as deep or long as the diameter of the cylindrical section 31. Opposed flanges 35 and 37 are formed in the arcuate strip on opposite sides of the reduced throat section 29 of notch 27. The opposed flanges 35 and 37 generally extend across an upper surface of a tube 2 positioned in the cylindrical section 31 of the notch 27 as generally shown in Figure 8.

The base 14 of the adaptor 10 preferably has a layer of adhesive applied thereto which is sufficiently tacky to hold or adhere the adaptor 10 to skin, but with sufficient release characteristics to allow the adaptor 10 to be readily removed from the wearer's skin and repositioned and secured in place. A backing sheet 41 is applied over the adhesive layer to protect the adhesive until the backing sheet 41 is removed. The backing sheet 41 covers the entire base14 and preferably includes a grasping tab 42 to facilitate separation of the backing sheet 41 from the adhesive layer on the base 14.

An alternative embodiment of the adaptor is shown in Figures 11-13 as tube seal adaptor 51. Tube seal adaptor 51 is of the same general shape as adaptor 10, except that its notch, notch 52, has straight sidewalls 55 spaced apart a uniform distance which extend to a curved or radiused bottom 57. As noted above, the notch 52 is at least as deep as it is wide and is preferably twice as deep as it is wide. An adaptor 51 of the configuration shown in Figures 11 and 12 is a preferred configuration when the adaptor is to be formed from a hydrogel which includes a synthetic polymeric matrix, water and a water soluble humectant. It is foreseen that tube seal adaptors 10 could also be formed form a hydrogel. Hydrogels of the type discussed above are described in Stout U.S. Pat. No. 4,671,267 which is incorporated herein by reference. U.S. Patent No. 6,082,360 describes use of such hydrogels to form seals for respiratory masks.

The hydrogel must be usable in conjunction with skin tissue without causing damage or injury to the skin. The hydrogels which are most suitable for use in conjunction with the present invention are non flowing and have memory, that is, the hydrogels and tube seal adaptors 10 or 51 made from the hydrogels return to a preformed shape when forces are removed from the tube seal adaptors 10 or 51 and when portions of the tube seal adaptors are not stuck to themselves.

Another property of the hydrogel seals is that such seals are sticky or tacky and have a strong affinity for both skin tissue and for the material construction of the mask 6, preferably silicon. The hydrogels of the tube seal adaptor 10 or 51 are self-sustaining and are compressible when pressure is applied against the seal 10 or 51 such that the seal fully conforms to and completely fills the space between the mask 6 and the face 4 and around the tube 2 when the mask 6 is applied to the face 4 of the user.

A hydrogel adaptor 51 is formed by pouring the hydrogel material into a mold and allowing it to cross-link. The surfaces of the cured hydrogel are sufficiently tacky to stick to a patient's skin, but readily releasable therefrom. It is foreseen that adaptors of the configuration shown in either Figures 2-8 or 11-13 could also be formed from a thermoplastic elastomer with an adhesive backing or base as well as from a closed cell foam or hydrogel material. Possible thermoplastic elastomers which could be utilized include: polyolefin plastomers (POP); thermoplastic polyolefin elastomer (TPO); polyolefin elastomer (POE); styrenic block copolymers (S-TPE); polyurethanes (polyester or polyether based)(TPU); polyamides (PEBA); and olefinic vulcanizates (TPV).

Referring again to Figures 2 and 3, tube seal adaptors 10 are shown positioned for use in association with a oro-nasal mask 6. In Figure 2 a first adaptor 10 is shown in use in association with a tube 2 inserted in a patient's mouth, and a second adaptor 10 is shown in use in association with a tube 2 inserted in the patient's nose. The thickness of the adaptors 10 shown is exaggerated in Figure 2 to more dearly show the adaptors 10. As discussed above, the adaptors 10 are generally positioned at a location at which a sealing flange or member 7 on the mask will extend across and engage the outer surface 16 of the adaptor 10 when the mask is held in place on the face 4 of a wearer. If only one tube 2 is used, usually only one adaptor 10 will be used. The adaptors 51 are used in the same manner.

The adaptors 10 will generally be used with a mask 6 using straps 61 or the like to pull the mask 6 and flange 7 against the wearer's face 4 and against any tube seal adaptors 10 positioned between the mask 6 and the wearer's face 4 as generally shown in Figure 3. The compressive force of the mask flange 7 being pulled against a tube seal adaptor10 generally compresses the adaptor 10 around the tube 2 to prevent air leaks along the tube 2. If the adaptor 10 selected for use with the existing tube 2 is sized so that the notch 27 is approximately twice as deep as the tube 2 is wide, there will be sufficient material of the adaptor 10 extending above the tube 2 which will be pressed together in abutting relationship across the upper surface of the tube 2 when the mask is pressed against the adaptor 10. The tube seal adaptor 51 will seal around a tube 2 in a similar manner when a mask is pressed against the adaptor 51.

Referring to Figure 13, if the tube 2 inserted in the notch 52 of adaptor 51 has a diameter which is about the same as the depth of the notch 52, the edges 55 of the notch 52 will not be pressed together in abutting relationship over the tube 2, but will be pressed against the tube 2 to form a generally smooth continuous surface across which the mask flange 7 can form a seal that does not leak. As long as at least half of the tube 2 extends into the notch 52 in the adaptor 51, the mask flange 7 should be able to form a leakproof seal around the tube 2. It is noted that the tube seal adaptor 10 will seal around a tube 2 in a similar manner when a mask is pressed against the adaptor 10.

Referring to Figures 9 and 10, a tube seal adaptor 10 is shown attached to a patient's face for maintaining a seal between a nasal mask 70 with a tube 2 inserted therebetween. The mask does include straps 71 for pulling the nasal mask 70 against the adaptor 10. The adaptor 10 is positioned generally on the upper lip of the wearer with the notch 27 aligned with a nostril into which the nasal tube 2 extends. The nasal mask 70 is then positioned so that an inner flange 72 on the nasal mask 70 extends across the adaptor 10.

It is to be noted that the outer surface 16 of an adaptor 10 or 51 formed from a hydrogel will be tacky and tend to stick to the mask flange 7. Although this may enhance the seal therebetween, the adaptor 10 or 51 may adhere to the mask 6 when removed from the wearer's face, requiring the adaptor 10 or 51 to be repositioned prior to subsequent use of the mask. In addition, if the adaptor 10 or 51 sticks to the mask, it increases the risk that the tube 2 will be withdrawn from the patient's nose or mouth if the adaptor 10 or 51 holding the tube 2 is pulled away from the wearer's face 4 with the mask 6. It is foreseen that a user could reduce the tackiness of the outer surface 16 of an adaptor 10 or 51 formed from hydrogel, by applying a coating of a non-adhesive material, such as an absorbent powder thereto. The outer surface 16 of the adaptor 10 or 51 formed from a closed cell foam, preferably does not have an adhesive applied thereto to facilitate separation of the mask 6 from the adaptor 10.

It is to be understood that while certain forms of the present invention have been illustrated and described herein, it is not to be limited to the specific forms or arrangement of parts described and shown.

## Claims

1. A tube seal adaptor for use in preventing leaks around a tube inserted between a respiratory mask and a face of a wearer of the respiratory mask comprising:
a) an arcuate strip of pliable material having a base, a convex outer surface, a first arcuate side, a second arcuate side, a first end and a second end;
b) a notch extending into said arcuate strip from said convex outer surface and extending between said first and second arcuate sides;
c) said convex outer surface converging toward said base at said first and second ends; and
d) said notch adapted to receive a tube and having a depth which is at least half as long as its width.

2. The tube seal adaptor as in Claim 1 wherein said notch is at least as deep as it is wide.

3. The tube seal adaptor as in Claim 1 wherein said notch is approximately twice as deep as it is wide.

4. The tube seal adaptor as in Claim 1 wherein said base is tacky.

5. The tube seal adaptor as in Claim 1 wherein-said notch includes a throat which opens into a cylindrical groove, said cylindrical groove having a diameter approximately equal to a diameter of an oro-nasal tube with which it is adapted for use, and said distance between opposed edges of said strip along said throat being smaller than the diameter of said cylindrical groove.

6. The tube seal adaptor as in Claim 1 wherein said pliable material forming said arcuate strip is sufficiently pliable and said notch is sufficiently deep such that opposed edges of said strip along portions of said notch extending outward from a tube seated in said notch are compressed into abutting relationship across the tube when a respiratory mask is drawn against said tube seal adaptor in seating relationship.

7. The tube seal adaptor as in Claim 1 wherein said adaptor is formed from a dosed cell foam.

8. The tube seal adaptor as in Claim 1 wherein said adaptor is formed from a hydrogel.

9. The tube seal adaptor as in Claim 1 wherein said adaptor is formed from a thermoplastic elastomer.

10. The tube seal adaptor as in Claim 1 in combination with a respiratory mask.

11. A tube seal adaptor in combination with a respiratory mask, said tube seal adaptor for use in preventing leaks around a tube inserted between said respiratory mask and a face of a wearer of said respiratory mask and comprising:
a) an arcuate strip of pliable material having a base, a convex outer surface, a first arcuate side, a second arcuate side, a first end and a second end;
b) a notch extending into said arcuate strip from said convex outer surface and extending between said first and second arcuate sides;
c) said convex outer surface converging toward said base at said first and second ends; and
d) wherein said notch is at least as deep as it is wide; and
e) said base is tacky.

12. The tube seal adaptor as in Claim 11 wherein said notch is approximately twice as deep as it is wide.

13. The tube seal adaptor as in Claim 11 wherein said base is tacky.

14. The tube seal adaptor as in Claim 11 wherein said notch includes a throat which opens into a cylindrical groove, said cylindrical groove having a diameter approximately equal to a diameter of an oro-nasal tube with which it is adapted for use, and said distance between opposed edges of said strip along said throat being smaller than the diameter of said cylindrical groove.

15. The tube seal adaptor as in Claim 11 wherein said pliable material forming said arcuate strip is sufficiently pliable and said notch is sufficiently deep such that opposed edges of said strip along portions of said notch extending outward from a tube seated in said notch are compressed into abutting relationship across the tube when a respiratory mask is drawn against said tube seal adaptor in seating relationship.

16. The tube seal adaptor as in Claim 11 wherein said adaptor is formed from a dosed cell foam.

17. The tube seal adaptor as in Claim 11 wherein said adaptor is formed from a hydrogel.

18. The tube seal adaptor as in Claim 11 wherein said adaptor is formed from a thermoplastic elastomer.

19. A tube seal adaptor in combination with a respiratory mask, said tube seal adaptor adapted for use in preventing leaks around a tube inserted between said respiratory mask and a face of a wearer of said respiratory mask and comprising a patch of pliable material having a base and a convex outer surface with a notch extending into said patch from said convex outer surface; said convex outer surface converging toward said base said base being sufficiently tacky to permit said patch to adhere to the face of a wearer of the respiratory mask; said notch adapted to receive a tube and having a depth which is at least half as long as its width.
